# EUROPEAN PATENT APPLICATION

(11) **EP 4 799 711 A1**
(43) Date of publication of application: **02.09.2026**
(21) Application number: 24882975.6
(22) Date of filing: 24.09.2024
(51) Int. Cl.: B01D 3/28, B01D 1/06, C07C 263/20, C07C 209/86

(54) **DEVICE AND METHOD FOR SEPARATING A MIXTURE CONTAINING THERMOLABILE SUBSTANCES**

(30) Priority: 23.10.2023 RU 2023127088
(71) Applicant: Public Joint Stock Company "Sibur Holding", Tyumenskaya oblast, 626150 (RU)
(72) Inventor: PETROV, Aleksandr Evgenevich, Uglich, 152610 (RU)
(74) Representative: Sloboshanin, Sergej
(86) International application number: PCT/RU2024/050225
(87) International publication number: WO 2025/089988

(57) **Abstract**

A device and method based on this device are proposed for continuous purification of high-boiling thermolabile compounds from more volatile impurities by selective continuous distillation of the medium fed into the device. The device is a column apparatus comprising at least three sections arranged in series: a mass transfer section, an evaporating section, and a cooling section, wherein the evaporating section is a vertically oriented gravity-flowing film evaporator with a fixed residence time of a thermolabile mixture in the evaporator zone. **In** a particular case, examples of high-boiling thermolabile media are mixtures of aromatic polyamines, for example, mixtures of methylenediphenylamine (MDA) and its higher homologues; as well as mixtures of aromatic polyisocyanates, for example, mixtures of methylenediphenylisocyanate (MDI) and its higher homologues. The method based on the device makes it possible to obtain the above-mentioned purified media with a residual content of more volatile solvents, such as aniline or chlorinated benzene, of no higher than a few tens of ppm, while the residence time of the medium being evaporated in the evaporator area is no longer than 100 seconds, which does not lead to significant changes in the chromaticity of the thermolabile medium

## Description

### Field of the invention

The invention relates to a device for the separation of a mixture containing at least one thermolabile substance. In particular, the invention relates to a column-type device consisting of at least three consecutive mass-transfer, evaporating and cooling sections, which are located one above the other and which are in fluid connection. The invention also relates to a method for the separation of a mixture containing at least one thermolabile substance, in particular to the method for the separation of polyamines and polyisocyanates. In particular, a device and method for the continuous purification of high-boiling thermolabile compounds by removing more volatile impurities using selective continuous distillation of the medium fed into the device are proposed.

### Prior art

Separation of mixtures containing thermolabile substances, i.e. substances that are characterized by thermal destruction, is one of the widely used processes in industry. More particularly, both the processes of separation, i.e. production of two or more different products, directly and the processes of purification, i.e. removal of undesirable impurities from the starting material, are used in industry. Hereinafter, the term "separation" includes, *inter alia*, purification processes. Examples of the processes of the separation of thermolabile mixtures are: separation of water-glycol solutions, separation of high-boiling aromatic compounds and pharmacological substances, purification of polyamines and polyisocyanates from unreacted starting compounds or solvents. It is precisely the sensitivity of thermolabile substances to temperature changes that results in a number of limitations associated with the design features of the devices used and their modes of operation.

According to the prior art, most processes of media separation by the method of continuous distillation are carried out using evaporators, the design of which assumes that the evaporating medium is subjected to prolonged heating and residence in the evaporator zone and outside its perimeter, with significant temperature loads. Examples of such evaporators include kettle-type evaporators or waste-heat boilers, vertical tube volume boiling evaporators, and thermosyphons. The enumerated evaporators, in combination with the volume of the space in which the bottom product accumulates in the lower section of the distillation column, result in the exposure of high-boiling media to temperature loads for the periods from tens of minutes to several hours.

One of the key representatives of thermolabile media are mixtures of polyamines and polyisocyanates. Raw (poly)diaminodiphenylmethane (pMDA) is an unrefined mixture of di- and polyamines, obtained as a result of the synthesis of di- and polyamines by condensation of aniline and formaldehyde. Subsequently, pMDA is used as a starting material for the production of polyisocyanates (pMDI) by phosgenation of pMDA. Said media are very sensitive even to low temperature loads that do not exceed 200-250^{°}C, and undergo irreversible structural changes associated with the processes of thermal destruction and resinification under prolonged heating. The absence of water in the initial pMDA is critical for carrying out the phosgenation step, because water promotes formation of a corrosive medium and reduces equipment lifespan, as well as the absence of aniline, because aniline contributes to the accumulation of phenylisocyanate, which is difficult to separate from the mixture of di- and polyisocyanates and acts as a poison for subsequent processes of the production of a polyurethane composition.

A volatile component of a thermolabile mixture to be distilled may be a component with a higher volatility relative to the thermolabile component nearest in volatility under the conditions of the distillation being conducted. The most common thermolabile media, such as pMDA and pMDI mixtures, contain the volatile components aniline and mono- or di-chlorobenzene, respectively, having a relative volatility in mixtures of more than 3 units at a residual pressure of less than 20 mbar.

Thus, US7312362 (published 25.12.2007 Bayer MaterialScience AG [DE]) discloses various variations of processes of the separation of di- and polyamines using distillation. The document proposes performing the final purification of the target components from aniline and water in a column-type apparatus, where the use of live steam as a heating agent is proposed. Separation processes include pre-evaporation, distillation and cooling. The disadvantages of this method are a high final proportion of water and aniline impurities in the target product - more than 100 ppm of water, which, as noted above, adversely affects the process of the subsequent phosgenation of the amine medium.

In WO2019115213 (published 20.06.2019 Sulzer Chemtech [CH]) a process and device for the distillation of a highly thermolabile mixture of di- and polyisocyanates are described. Thus, according to the proposed solution, the distillation column evaporator is a falling film evaporator. However, the implementation of this method requires forced circulation of the bottom product and a long residence time of the medium which accumulates in the lower section of the column at its boiling point.

The authors of DE202013003950 (published 25.06.2013 Huntsman LLC) propose performing the final purification of pMDA in a column-type apparatus, using additional stripping of volatile components with a heated nitrogen stream. This solution is characterized by all the above disadvantages, and also, in addition, the load on the vacuum-generating unit during pumping of stripping gas is increased.

Thus, the problem of developing a method for the separation of mixtures containing thermolabile substances, which makes it possible to achieve a high degree of purity of separation products in the absence of the detrimental process of thermal decomposition, remains relevant.

### Summary of the invention

**The problem to be solved by the present invention** is to provide a process for the separation of mixtures containing high-boiling thermolabile substances, which is characterized by the absence of the detrimental process of thermal decomposition.

**The technical result** is to provide the separation products containing high-boiling thermolabile substances with a residual content of volatile substances of no more than 100 ppm and a minor change in the initial chromaticity.

**This technical problem is solved and the technical result is achieved** by means of the use, in the process for the separation of mixtures containing thermolabile substances, of a device which comprises a mass transfer section with an inlet for the mixture, an evaporating section located under the mass transfer section and in fluid connection with it, and a cooling section located under the evaporating section and in fluid connection with it with an outlet for the liquid stream containing a thermolabile substance, wherein the evaporating section contains a vertical film evaporator with a distribution unit located in its upper part for the formation of a film of the mixture coming from the mass transfer section on the inner surface of the vertical film evaporator, wherein the vertical film evaporator is configured to heat the film, thus generating a vapour phase of the mixture and a liquid stream containing the thermolabile substance, wherein the distributing unit is further configured to allow the vapour phase of the mixture to pass through it from the vertical film evaporator to the mass transfer section, wherein the mass transfer section is configured to provide contact between the mixture fed into the mass transfer section and the vapour phase of the mixture coming from the evaporating section, wherein the vertical film evaporator is configured to transfer the liquid stream containing the thermolabile substance from the evaporating section to the cooling section for cooling thereof.

Without wishing to be bound by a particular theory, the author of the present invention believes that the use of the device described above reduces the residence time of thermolabile substances in the zone of elevated temperatures in the evaporating section, which makes it possible to avoid undesirable processes of the decomposition thereof, and at the same time to achieve a low content of undesirable volatile impurities (water, unreacted initial compounds, reaction by-products, etc.) in the separation product.

Effective separation of mixtures containing high-boiling thermolabile substances, characterized by the absence of a detrimental process of thermal decomposition, is achieved by the successive arrangement of the three key sections - the mass transfer section, in which the input mixture undergoes separation by means of mass transfer between the liquid and vapour phases of the mixture, the evaporating section, in which the vapour phase is generated from the film of thermolabile liquid, which is then fed into the mass transfer section, and the cooling section, which limits the time of the exposure of the medium to high temperatures after the evaporating section.

Effective separation of mixtures is achieved due to mass transfer processes occurring in the mass transfer section of the device between the downstream flow of the input liquid fed into the device (irrigation flow in the upper section of the mass transfer section) and the upstream flow of the saturated vapour generated in the evaporating section. Besides, a vertically oriented film evaporator in the evaporating section provides a gravity-driven flow of film along its inner surface which provides a residence time of thermolabile substances in the high-temperature zone that avoids their thermal decomposition. For amine and isocyanate media, the residence time of the medium in the evaporating section does not exceed 100 seconds.

According to one embodiment of the present invention the film evaporator comprises vertically oriented tubes. Preferably the tube length is: $L \left[m\right] = τ _ Film * wcp ,$ where τ_Film is the average residence time of the film stream on the inner surface of the heat exchanger tubes, said residence time ranging from 1 to 100 seconds, wcp is the average velocity of gravity-driven flow of the film along the inner surface of the tubes.

Preferably, the tube length is chosen to provide a residence time of the flow of film τ_Film of 20 to 80 seconds. More preferably, the tube length is chosen to provide a residence time of the film stream τ_Film of 30 to 60 seconds.

Moreover, the distributing unit is configured to form a film on the inner surface of the tubes of the film evaporator.

According to one embodiment of the present invention, the distributing unit intended for forming, on the inner surface of the vertical film evaporator, a film of the mixture entering from the mass transfer section, is a draw-off tray.

The device according to the present invention preferably may be used to separate a mixture of di- or polyamines or di- or polyisocyanates.

Preferably, the mass transfer section is a packed column with an internal contact unit based on mesh or sheet structured packing elements.

Preferably, the internal contact unit has a specific surface area of 400-1000 m²/m³ and a void volume of more than 80%.

In the device according to the present invention, the cooling section is or comprises an inclined gravity-flowing tubular heat exchanger of the "tube in tube" type or a heat exchanger-recuperator. Preferably, the gravity-flowing tubular heat exchanger has an inclination angle of the heat exchanger axis to the overall horizontal position of the device of 1-90°.

Another aspect of the present invention, which provides for the achievement of the above technical result, is a method for the separation of a mixture containing at least one thermolabile substance using a device for the separation of said mixture, which comprises a mass transfer section, an evaporating section located under the mass transfer section and in fluid connection with it, containing a vertical film evaporator, and a cooling section located under the evaporating section and in fluid connection with it, wherein the mixture containing at least one thermolabile substance is fed into the mass transfer section, after which it enters the vertical film evaporator of the evaporating section, thereby forming a film, which is gravity-flowing along the inner surface of the film evaporator under the action of gravity, wherein the gravity-flowing film of the mixture is heated in the vertical film evaporator, thereby generating a vapour phase of the mixture and a liquid stream containing the thermolabile substance, wherein the vapour phase of the mixture is lifted into the mass transfer section for mass transfer via the contact between the vapour phase of the mixture and the mixture fed into the mass transfer section, wherein the liquid stream containing the thermolabile substance from the evaporating section is transferred to the cooling section, where it is cooled and drawn off as a separation product containing the thermolabile substance.

Preferably, the method is used for the separation of the mixture of di- or polyamines or di- or polyisocyanates. Preferably, the method is used to separate (poly)diaminodiphenylmethane (pMDA) or polydiaminodiphenylisocyanates (pMDI) to produce two or more streams of pMDA or pMDI.

According to the present invention, the residence time of the film stream in a vertical film evaporator is from 1 to 100 seconds.

### Description of figures

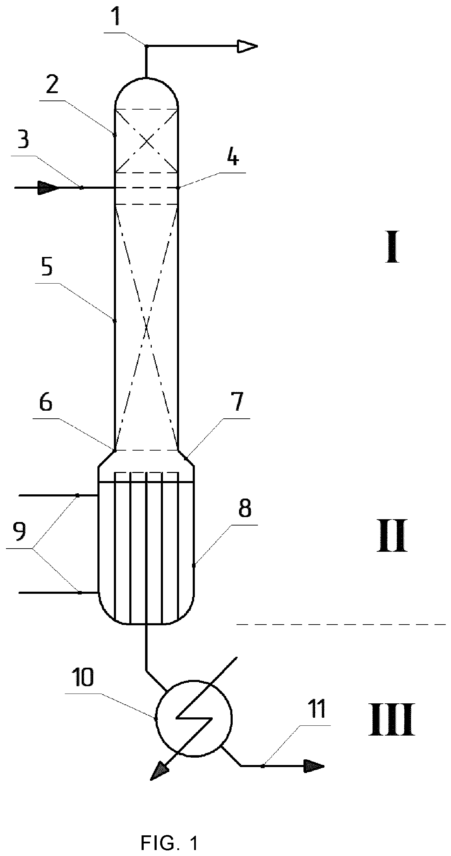

Fig. 1 shows the device according to the invention, wherein I - the mass transfer section, II - the evaporating section, III - the cooling section, 1 - a vapour phase outlet, 2 - an entrainment separator or a reinforcing section of the mass transfer part of the device, 3 - a mixture inlet for separation/purification, 4 - a collector-distributor of vapour-liquid streams, 5 - a contact unit, 6 - supporting elements, 7 - a distributing unit for vapour and liquid phases, 8 - a gravity-flowing vertically oriented film evaporator, 9 - a heat-transfer agent, 10 - a cooler, and 11 - an outlet for separation products.

### Detailed description of the invention

The main aspects of the present invention are described in detail below.

The device according to the present invention is a column-type device consisting of at least three consecutive sections: a mass transfer section (I), an evaporating section (II) and a cooling section (III).

The mass transfer section (I) of the column device is a section of the column, into which a mixture containing thermolabile substances is fed via the inlet 3 for separation and which is equipped with an internal contact unit 5 (ICI) and supporting elements 6 for its fastening. The contact units 5 of the mass transfer part can be selected from any contact units known from the art, including mass transfer trays, structured packing elements, etc. It is most preferable to use mesh or sheet structured packing elements as a contact unit with the specific surface area of 400-1000 m²/m³ and a void volume of more than 80% since this type of contact unit has an optimal number of separation stages per unit of the column height, as well as an acceptable hydraulic pressure drop in conditions of the traditional conduct of distillation processes at a low residual pressure.

The material design of the ICI and the optimal number of separation stages in the mass transfer part of the device are different depending on the physico-chemical properties and vapour-liquid equilibria of the components of the mixture to be separated. Thus, for example, the optimal number of stages for the separation of a mixture of polyamines with water and aniline or a mixture of polyisocyanates with a chloroaromatic solvent, for example, monochlorobenzene, ranges from 3 to 20, more preferably from 4 to 10, even more preferably from 5 to 8; and the optimal material for the ICI is austenitic stainless steels, for example, AISI304/316.

Optionally, in order to intensify and arrange optimal operation of the device, the mass transfer part of the column device can be equipped with an entrainment separator 2 and a collector-distributor 4. The entrainment separator 2 is any unit available for the purpose of capturing any entrained droplets, for example, a structured packing element with a specific surface area of 100-2000 m²/m³.

Optionally, the entrainment separator 2 can be replaced or modified in order to arrange a reinforcing section of the column and reduce the proportion of high-boiling substances in the top product stream. However, even without significant additional changes in the scenario when the device is fed with an amine or isocyanate medium, said section may have an affinity for mass transfer between trapped droplets and the raising vapour phase of the more volatile components of the medium.

The vapour phase can be drawn off via an outlet 1 located in the upper section of the mass transfer section.

The evaporation section (II) of the device is a gravity-flowing vertically oriented film evaporator 8 located below the mass transfer section. The film evaporator 8 is of a one-pass tube evaporator type which is close in geometric characteristics to tubular heat exchange apparatuses that meet TEMA international standards. The vapour phase generation for the mass transfer process occurs on the inner surface of the vertically oriented stack of tubes in a film mode. Unlike typical volume boiling devices, in which the vapour phase is generated in the volume of liquid in the form of gas bubbles, in the film mode, the equilibrium vapour phase is formed above the surface of a gradually heated thin film flowing along the vertical surface. In this case, the movement of the liquid and vapour phases from the evaporator is performed in counter-flow.

The gravity-flowing vertically oriented film evaporator 8 according to the present invention is a film evaporator without forced circulation of the mixture. Such a vertically oriented gravity-flowing film evaporator provides for the fixing of a residence time of a mixture containing thermolabile substances.

The evaporating section is equipped with a distributing unit 7, which is any available distributing unit in the described art that provides even distribution of the liquid phase on the inner surface of the evaporator or evaporator tubes. It is most preferable to use distributing units for direct irrigation of the wetted perimeter of the tubes, in which the liquid entering the evaporator wets the tubes due to the overflow under the action of gravitational forces.

The film mode in the vertical tubular evaporator 8 according to the invention is set arbitrarily at a certain irrigation density by means of the natural overflow of the liquid medium via the distributing unit 7 onto the inner surface of the evaporator 8, in particular onto the inner surface of the evaporator tubes.

In the evaporator 8 according to the invention, no forced means are used to create and/or maintain a film mode on the inner surface of the evaporator or of a stack of the evaporator tubes, such as, for example, pumps, manifolds, and such like. In the present invention precisely this particular feature is defined as self-flow. In the evaporator 8, no means are used, except for the natural barrier of the distributing unit 7, which makes it possible to provide sufficient irrigation density for the stability of the film mode.

One advantage of such an evaporator is the short residence time of the mixture containing thermolabile substances on the surface of the heat exchange tubes, which is limited by the time of gravity-driven flow of the film of liquid from the evaporator distribution element to the next section of the column apparatus, i.e. the cooling section. Another advantage of the described evaporator type is that there is no need for the use of an expensive high-temperature circulation pump, which is often used for the arrangement of the separation process, but, at the same time, is the most vulnerable zone for seal failure and contact of the mixture containing thermolabile and/or highly reactive substances with the atmospheric oxygen.

It is obvious to a person skilled in the art that specific geometric parameters of the described device depend on characteristics of the mixtures to be separated. To this end, it is useful to use the following set of formulae for describing the geometric characteristics of the evaporating part of the device: $Re = \left(4*Gop\left[kg/\left(m*s\right)\right]\right) / \left(μ\left[Pa*s\right]\right) ,$
wherein Re is the Reynolds number; Gop is the irrigation density calculated using formula (2); µ is the dynamic viscosity of the mixture to be separated, which is passing through the evaporator. $G_op = \left(G\left(N-1 liq\right)\left[kg/\left(m*s\right)\right]\right) / \left(\sum \left〚P tube\right〛 \left[m\right]\right) ,$
wherein G (N-1 liq) is the amount of liquid used for irrigation of the evaporator; Ptube is the inner perimeter of the wetted heat exchange tube of the evaporator. $δcp = √ 3 \left(\left(3*Gop*μ\right)/\left({ρ}^{∧}2*g\right)\right) \left[m\right] ,$
wherein cp is the average thickness of the film of liquid formed on the inner surface of the heat exchange tubes, ρ is the density of the medium to be separated, which is passing through the evaporator (kg/m³); g=9,8 is gravitational acceleration. $w_aver = √ 3 \left(\left({Gop}^{2}*g\right)/\left(3*μ*ρ\right)\right) ,$
wherein w_aver is the average velocity of gravity-driven flow of the film along the inner surface of the tubes [m/s]. $τ_Film = \left(L\left[m\right]\right) / wcp ,$ wherein L is the length of the heat exchange tubes of the film evaporator, τ_Film is the average residence time of the liquid on the inner surface of the heat exchange tubes.

When a polyamine or polyisocyanate medium is fed into the device, the preferred range of the Reynolds number is 0.1-10000, more preferably 1-1000, even more preferably 1-50.

The tube length can be selected from the standard size range of TEMA heat exchange apparatuses of BEM type. When an amine or isocyanate medium is fed into the device, the preferred tube length is such that the time of gravity-flow of the film of liquid is in the range from 1 to 100 sec, preferably from 20 to 80 sec, more preferably from 30 to 60 sec.

The film evaporator of the proposed design can be heated by any available and effective heat-transfer agent 9, for example, by steam under appropriate pressure or by a high-boiling organic heat-transfer agent, for example, organosilicon heat-transfer agents or polyalkylbenzene-based heat-transfer agents, which have suitable thermophysical characteristics.

The third section (III) of the device is the cooler 10 for the stream containing thermolabile substances, which exits the lower section of the film evaporator 8. The cooler 10 may be any available cooling device from the art, including, but not limited to: gravity-flowing inclined tubular heat exchangers, heat exchangers-recuperators. A suitable example of such a device may be an inclined tubular heat exchanger of the "tube in tube" type, with an axis inclination relative to the overall horizontal position in the range of 1-90 degrees, more preferably 15-60 degrees. The advantage of this section is that it limits the residence of the stream containing thermolabile substances at high temperature in the perimeter of the film evaporator 8, which reduces the residence time of the thermolabile medium at high temperature down to the time which the gravity-flowing film spends on the inner surface of the evaporator 8 tubes. Therefore, the risk of thermal destruction of thermolabile substances is minimized and, accordingly, the physical and mechanical properties and chromaticity index of the medium are maintained.

The stream exiting from the bottom of the device can be directed, as a feedstock, to further chemical processing or directed to be worked up into a commercial product.

Vapours accumulated at the top of the device can be condensed by any method known from the art. The most preferred condensation method is the method of the vapor phase condensation on the outer surface of a horizontally oriented stack of tubes of the shell-tube heat exchanger. The advantage of such a method is the low hydraulic pressure drop in the vapour phase condensation system.

A specific example of separation of a mixture containing thermolabile substances is the separation of di- and polyamines, water and aniline. This description is given as an example only and is not limiting, as is obvious to a person skilled in the art.

Preferably, a feedstock mixture of di- and polyamines (pMDA) mixed with water and aniline is fed into the device (inlet 3). The water proportion in the pMDA feedstock is 0-15 wt.%, more preferably 0-8 wt.%. In order to reduce the steam load in the mass transfer section (I) of the device, it is possible to pre-dehydrate the pMDA feedstock using any technique available in the art. The most preferred method for pre-dehydration is one-step evaporation of the mixture in an evaporator-separator (flash), where, in mild conditions at a temperature of up to 150^{°}C and a pressure of not lower than 200 mbar, a significant proportion of water can be removed from the mixture in the form of evaporated water-aniline azeotrope. The aniline proportion in the feedstock mixture can be 0-60 wt.%, more preferably 0-30 wt.%. The process of distillation of the di- and polyamines mixture is performed at a residual pressure in the range of 1-50 mbar, more preferably 2-20 mbar, even more preferably 3-10 mbar. The temperatures of the top and bottom sections of the mass transfer section (I) are set in accordance with the oligomeric composition of polyamines, the temperature of the inlet feedstock stream and the requirement for the removal of the components of the oligomeric composition. The temperature in the evaporating section (II), depending on the oligomeric composition of the polyamines, is 200-280°C, more preferably 220-250°C. The temperature of the cooling section (III) is set to be sufficient to cool the amine medium to a temperature of no higher than 150^{°}C, more preferably no higher than 100-120^{°}C. Due to a high viscosity of the resulting medium, the amine medium is stored at a temperature of no lower than 60-100^{°}C prior to final dispatch.

The residual content of volatile components achieved is individual to the specific thermolabile media and components extracted from them. More specifically, the capabilities of the device are disclosed in the examples based on distillation of thermolabile media such as pMDA and pMDI in a mixture with aniline and monochlorobenzene, respectively.

The improvement achieved in the chromaticity and viscosity of thermolabile products, which are drawn off from the bottom section of the device (via an outlet 11), is individual to the specific thermolabile medium fed into the device. Due to the fact that the described characteristics of the thermolabile media are a function with time under specific temperature stresses, it is obvious that the described device with a short contact residence time makes it possible to avoid to a greater extent the undesirable impact of thermal destruction.

More specifically, the capabilities of the device are disclosed in the examples based on distillation of thermolabile media such as pMDA and pMDI in a mixture with aniline and monochlorobenzene, respectively.

### Examples of the embodiment of the invention

### Analysis methods:

1. The mass proportions of oligomers and aniline in the samples of pMDA were determined by the liquid chromatography (HPLC) method on the liquid chromatograph Agilent 1260 Infinity II, equipped with diode array detector 1260 DAD WR and chromatographic column Kinetex C18, 250 × 4.6, 5 µm, 100 Å. The samples were preliminary dissolved in acetonitrile with added DMSO. The calculation was carried out using the absolute calibration method.
2. The residual water content in the samples of pMDA was determined by the coulometric titration method according to Karl Fischer on the automatic titrator according to GOST 24614 "Liquid and gases which do not react with Fischer reagent. The coulometric method of water determination." The following reagents were used to determine water: Hydranal-Coulomat AK and Hydranal-Coulomat CK.
3. The mass proportion of water in aniline was determined by the gas chromatography (GC) method using the gas chromatographic appliance Agilent 7890A, equipped with a thermal conductivity detector and DB-FFAP capillary column (30m × 0.32mm × 0.25µm). The calculation of the mass proportion of water was carried out using the absolute calibration method.
4. The viscosity of the pMDA was determined using an Anton Paar MCR 102 modular compact rheometer equipped with a measuring system PP 25 (plate/plate type), based on the measurement of the moment of resistance to the rotation of a plate of the measuring appliance of the tested sample at different rotation speeds (shear speeds) and the calculation of shear stress and dynamic viscosity.
5. The chromaticity of the pMDA and pMDI samples was determined using Lovibond PFXi-995 spectrophotometric colorimeter and a cell made of pre-stressed optical glass with an optical path length of 10 mm. The method is based on the measurement of colour and chromaticity coordinates of a sample diluted with solvent (ethyl acetate for pMDA, monochlorobenzene for pMDI; 20-25 wt.%) in the wavelength range from 420 to 710 nm. The result is given in colour units of the iodine scale.
6. Mass proportions of oligomers in pMDI samples were determined by the HPLC method with preliminary derivatization of samples with methanol and subsequent analysis of the resulting reaction mass on an Agilent 1260 Infinity II liquid chromatograph equipped with a diode array detector 1260 DAD WR and Kinetex chromatographic column C18, 250 × 4.6, 5 µm, 100 Å. The oligomer content in the samples was determined by the method of internal normalization of peak areas.
7. Mass proportions of phenylisocyanate and 4,4'- methylenediphenyldiisocyanate impurities in the samples of MCB distillate were determined by the GC method on an Agilent 7890A gas chromatograph equipped with a flame-ionization detector and DB-35 capillary column (30 m × 0.25 mm × 0.25 µm). The calculation was carried out using the absolute calibration method.
8. Dynamic viscosity of the pMDI was calculated using kinematic viscosity and density.
   8.1 Kinematic viscosity was determined using capillary viscometers of VPZh-1 type. The determination consists in measuring the flow time, in seconds, of a certain volume of the tested fluid under the influence of gravity at a constant temperature, using a calibrated glass viscometer. Kinematic viscosity is the multiplication of the measured flow time and viscometer constant.
   8.2 pMDI density was determined at a temperature of 25°C according to GOST 18995.1 "Chemical liquid products. Methods of Density Determination."

### Example 1 (according to the invention)

The device according to Fig. 1 consisted of a mass transfer section consisting of an AISI316 stainless steel tube with the internal diameter of 50 mm, which was filled with Sulzer CY structured packing elements having a total packed height of 0.8 m; an entrainment separator section comprising AISI316 stainless steel tube with an internal diameter of 50 mm, which was filled with Sulzer CY structured packing elements having a total packed height of 160 mm; a multi-point liquid distributor with a specific distribution capacity of about 1000 points/m². In order to minimize thermal losses, the local zones of the column body were heated by electric heating to a temperature of about 200^{°}C and insulated with mineral wool.

The device according to Fig. 1 consisted of an evaporating section, which is a vertical shell-tube heat exchanger with an internal diameter of the shell of 80 mm and three heat exchange tubes 28x1.5 mm 400 mm long, heated by Thermolan Lab5 hot silicone oil being fed. The distributing unit was a draw-off tray, and the film was created by overflowing liquid through the wetted perimeter of the tubes. The calculated hydrodynamic mode of the flow of film was characterized by the Reynolds number of about 30 and the pMDA residence time of about 30 sec.

The device according to Fig. 1 consisted of a cooling section, which is an inclined gravity-flowing heat exchanger of the "tube in tube" type with an inner diameter of 25 mm of the inner tube and a total inclination relative to the base plane of the column of 105°, PMS10 silicone oil was fed into the tube annulus.

pMDA raw material for testing the device was obtained according to the method described in Pat. CN100422239. The initial chromaticity of the pMDA raw material was 23 units on an iodine scale. The pMDA raw material fed into the device had the following average composition: water proportion 0.25-0.5 wt.%, aniline proportion 16.33 wt.%, the mixture of MDA and its oligomers - the remainder. The raw materials was fed with the flow rate 1.1-1.15 kg/h, and the temperature of the input stream was maintained by electric heating at 80°C.

In the top section of the mass transfer section of the device, the residual pressure of 6-8 mbar was maintained, and in the evaporating section the temperature of the heat-transfer agent was maintained at 245-250^{°}C. The pressure drop between the top section of the mass transfer section to the bottom of the evaporating section was less than 1.5 mbar. The temperature of the leaving vapours was 55-65^{°}C, and the vapour temperature at the bottom section was about 235^{°}C.

Once the pMDA stream left the evaporator at the amount of 950-1000 g/h it was cooled in the cooling section to a temperature of 100-120^{°}C, and then collected in a 12 L receiving container equipped with external electric heating.

The drawn-off vapour phase in an amount of 100-200 g/h was condensed in the shell-tube heat exchanger-condenser on the outer surface of the stack of finned tubes with a specific surface of about 1 m². A water-glycol heat-transfer agent with a temperature of 10-25°C was fed into the tube space of the condenser. The condensed aniline fraction was collected in a 12 L receiving container equipped with an external jacket, in the annulus of which the water-glycol heat-transfer agent was directed after the vapour condenser.

The accumulated fractions of the top and bottom products were unloaded periodically every 4-6 h of continuous operation of the device, and then samples were taken from the fractions for the conduct of quantitative and qualitative analysis by the HPLC method.

The condensate fraction, which consists mainly of a mixture of aniline and water, contained up to 0.001 wt.% MDA. The bottom product of the device, purified pMDA, contained less than 10 ppm of water and less than 20 ppm of aniline. The chromaticity of the purified pMDA was 43 units on an iodine scale. The viscosity of the purified pMDA was 34 mPa*sec at 100°C.

### Example 2. (according to the invention)

The device described in example 1 was used to extract, from the stream of 950-980 g/h of pMDA, the following average composition: water proportion less than 10 ppm, aniline proportion less than 40 ppm, MDA proportion 48 wt.%, higher oligomers of the MDA - the remainder; MDA flow in the amount of 2-25% of the mass feed flow of the device. The chromaticity of the pMDA feeding stream was 43 units on an iodine scale. The temperature of the feeding stream was maintained at 150-200^{°}C using electric heating. The calculated hydrodynamic mode of the film evaporator was characterized by the Reynolds number of about 20 and the pMDA residence time of about 30 sec.

In the top section of the mass transfer section of the device, the residual pressure of 3-4 mbar was maintained, and in the evaporating section the temperature of the heat-transfer agent was maintained at 225-235^{°}C. The pressure drop between the top section of the mass transfer section to the bottom of the evaporating section was less than 1.5 mbar. The temperature of the leaving vapours was 200-210^{°}C, and the vapour temperature at the bottom section was 220-230^{°}C.

Once the pMDA stream left the evaporator in an amount of 850-950 g/h it was cooled in the cooling section to a temperature of 120^{°}C, and then collected in a 12 L receiving container equipped with external electric heating.

The drawn-off vapour phase in an amount of 50-150 g/h was condensed in the shell-tube heat exchanger-condenser on the outer surface of the stack of finned tubes with a specific surface of about 1 m². A organosilicon heat-transfer agent with a temperature of 110°C was fed into the tube space of the condenser. The condensed fraction was collected in a 12 L receiving container equipped with an external jacket, in the annulus of which the organosilicon heat-transfer agent was directed after the vapour condenser.

Depending on the temperature of the inlet stream and the heat load on the evaporator, the proportion of the MDA recovered from the inlet stream was 2-25 wt.% of the total amount of the feedstock stream.

The accumulated fractions of the top and bottom products were unloaded periodically every 4-6 h of continuous operation of the device, and then samples were taken from the fractions for the conduct of quantitative and qualitative analysis by the HPLC method.

The condensate fraction, which is a melted MDA, contained up to 0.3 wt.% M3A. The bottom product of the device, purified pMDA with a reduced MDA proportion, contained less than 1 ppm of water and less than 10 ppm of aniline. The pMDA colour change following the MDA distillation was less than 10 units on the iodine scale. The viscosity of the pMDA was 48 mPa*sec at 100°C.

### Example 3. (according to the invention)

The purified pMDA produced in example 2 was phosgenated according to the method described in WO2023/063852. Following the phosgenation of the isocyanate medium and its preliminary purification by filtration, the resulting product was directed to the column apparatus described in example 1 for the recovery of the excess solvent from the feedstock stream.

Thus, 1100-1150 g/h of raw pMDI of the following average composition was fed into the device: the monochlorobenzene proportion - 10-15 wt.%, the pMDI proportion - the remainder. The temperature of the feeding stream was maintained at 50-80^{°}C using electric heating. The calculated hydrodynamic mode of the film evaporator was characterized by the Reynolds number of about 20 and the pMDI residence time of about 30 sec.

In the top section of the mass transfer section of the device, the residual pressure of 5-10 mbar was maintained, and in the evaporating section the temperature of the heat-transfer agent was maintained at about 200°C. The pressure drop between the top section of the mass transfer section to the bottom of the evaporating section was less than 1.5 mbar. The temperature of the leaving vapours was 40-45°C, and the vapour temperature at the bottom section was up to 200°C.

Once the pMDI stream left the evaporator in an amount of 1000 g/h it was cooled in the cooling section to a temperature of 60-70°C, and then collected in a 12 L receiving container equipped with external electric heating.

The drawn-off vapour phase in an amount of 100-150 g/h was condensed in the shell-tube heat exchanger-condenser on the outer surface of the stack of finned tubes with a specific surface of about 1 m². A organosilicon heat-transfer agent with a temperature of -20°C was fed into the tube space of the condenser. The condensed fraction was collected in a 12 L receiving container equipped with an external jacket, in the annulus of which the organosilicon heat-transfer agent was directed after the vapour condenser.

The accumulated fractions of the top and bottom products were unloaded periodically every 4-6 h of continuous operation of the device, and then samples were taken from the fractions for the conduct of quantitative and qualitative analysis by the HPLC method.

The condensate fraction, which is an MCB, contained trace amounts of MDI isomers. The bottom product of the device, purified pMDI, contained less than 30 ppm of MCB. The pMDI chromaticity was not higher than 110 units on the iodine scale, and the pMDI viscosity was 180 mPa*sec at 25°C.

### Example 4. (Comparative)

Raw pMDA, produced according to the method described in Pat. CN100422239, and having the following average composition: water proportion - 0.25-0.5 wt.%, aniline proportion - 16.33 wt.%, the mixture of MDA and its oligomers - the remainder; it was subjected to a staged purification from water and aniline and to the recovery of the recycled MDA fraction in an amount of up to 25% of the initial mass of the polyamine medium, by a periodic fractional distillation in a device consisting of a laboratory 500 mL distillation retort equipped with a magnetic stirring appliance and placed in an electric flask heater and connected to a steam fraction receiver using the "trap to trap" method and to suitable air heaters and steam flow coolers.

The distillation process was performed at temperature and pressure settings close to the technological conditions of the processes described in examples 1 and 2 respectively. The bottom product residence time ranged from 45 min to 90 min while all the stages of the purification of the medium by distillation were carried out.

During fractionation of 150 g of the loaded raw pMDA, about 26 g of water-aniline fraction and about 13 g of MDA fraction were obtained. At the end of the fractionation, samples were collected from the top fractions and the bottom product for the conduct of quantitative and qualitative analysis by the HPLC method.

The bottom product obtained during fractionation, purified pMDA, contained less than 10 ppm of aniline and had a chromaticity of 66 units on an iodine scale and a viscosity of 56 mPa*sec at 100°C.

### Example 5. (Comparative)

The raw pMDI produced in example 3 was subjected to periodic fractionation in a device similar to the device described in comparative example 4, except that the distilling retort and the receiver of the distillate fraction were connected to each other via a direct condenser, to cool which a chilled organosilicon heat-transfer agent was supplied.

The distillation process was performed at temperature and pressure settings close to the technological conditions of the processes described in example 3. The residence time of the bottom product during fractionation ranged from 45 min to 90 min.

During fractionation of 150 g of the loaded raw pMDI, about 16 g of the MCB fraction were obtained. At the end of the fractionation, samples were collected from the top fraction and the bottom product for the conduct of quantitative and qualitative analysis by the HPLC method.

The bottom product obtained during fractionation, purified pMDI, contained less than 30 ppm of MCB and had a chromaticity of 305 units on an iodine scale and a viscosity of 233 mPa*sec at 25°C.

**Table 1. Summary table of example characteristics**

| Example | Type of thermolabile medium | Distillation method | Evaporator temperature, °C | Residence time, sec | Product viscosity, mPa*sec, at temp. in °C | Chromaticity of the bottom product, on an iodine scale |
|---|---|---|---|---|---|---|
| #1 | pMDA | According to the invention | Up to 250 | ∑ 30 | 34 (100°C) | 43 |
| #2 | pMDA | | | ∑ 30 | 48 (100°C) | 52 |
| #3 | pMDI | | Up to 200 | ∑ 30 | 180 (25°C) | 110 |
| Comparative #4 | pMDA | Periodic fractionation | Up to 250 | 2700-3600 | 56 (100°C) | 66 |
| Comparative #5 | pMDI | | Up to 200 | 1800-3600 | 233 (25°C) | 305 |

## Claims

1. Device for the separation of a mixture containing at least one thermolabile substance, wherein the device comprises:
a mass transfer section with an inlet for the mixture, an evaporating section located under the mass transfer section and in fluid connection with it, and a cooling section located under the evaporating section and in fluid connection with it with an outlet for the liquid stream containing a thermolabile substance, wherein the evaporating section contains a vertical film evaporator with a distributing unit located in its upper part for the formation of a film of the mixture coming from the mass transfer section is formed on the inner surface of the vertical film evaporator, wherein the vertical film evaporator is configured with ability to heat the film, thus generating a vapour phase of the mixture and a liquid stream containing the thermolabile substance, wherein the distributing unit is further configured to allow the vapour phase of the mixture to pass through it from the vertical film evaporator to the mass transfer section, wherein the mass transfer section is configured to provide contact between the mixture fed into the mass transfer section and the vapour phase of the mixture coming from the evaporating section, wherein the vertical film evaporator is configured to transfer the liquid stream containing the thermolabile substance from the evaporating section to the cooling section for cooling thereof.

2. Device according to Claim 1, wherein the film evaporator contains vertically oriented tubes.

3. Device according to Claim 2, wherein the length of the tubes is: $L \left[m\right] = τ _ Film * wcp ,$ where τ_Film is the average residence time of the film stream on the inner surface of the heat exchanger tubes, said residence time ranging from 1 to 100 seconds, wcp is the average velocity of gravity-driven flow of the film along the inner surface of the tubes.

4. Device according to Claim 3, wherein the length of the tubes is selected to provide for the residence time of the film stream τ_Film from 20 to 80 seconds.

5. Device according to Claim 4, wherein the length of the tubes is selected to provide for the residence time of the film stream τ_Film from 30 to 60 seconds.

6. Device according to Claim 2, wherein the distributing unit is configured to form a film on the inner surface of the tubes of the film evaporator.

7. Device according to Claim 1, wherein the distributing unit is a draw-off tray.

8. Device according to Claim 1, intended for the separation of a mixture of di- or polyamines or di- or polyisocyanates.

9. Device according to Claim 1, wherein the mass transfer section is a packed column with an internal contact unit based on mesh or sheet structured packing elements.

10. Device according to Claim 9, wherein the internal contact unit has a specific surface area of 400-1000 m²/m³ and a void volume of more than 80%.

11. Device according to Claim 1, wherein the cooling section is or includes an inclined gravity-flowing tubular heat exchanger of the "tube in tube" type or a heat exchanger-recuperator.

12. Device according to Claim 11, wherein the gravity-flowing tubular heat exchanger has an inclination angle of the heat exchanger axis to the overall horizontal position of the device of 1-90°.

13. Method for the separation of the mixture containing at least one thermolabile substance by means of the device for the separation of the mixture, the device comprising a mass transfer section, an evaporating section located under the mass transfer section and in fluid connection with it, which contains a vertical film evaporator, and a cooling section located under the evaporating section and in fluid connection with it,
wherein the mixture containing at least one thermolabile substance is fed into the mass transfer section, after which it enters the vertical film evaporator of the evaporating section, thereby forming a film, which is flowing along the inner surface of the film evaporator under the action of gravity, wherein the gravity-flowing film of the mixture is heated in the vertical film evaporator, thereby generating a vapour phase of the mixture and a liquid stream containing the thermolabile substance, wherein the vapour phase of the mixture is lifted into the mass transfer section for mass transfer via the contact between the vapour phase of the mixture and the mixture fed into the mass transfer section, wherein the liquid stream containing the thermolabile substance from the evaporating section is transferred to the cooling section, where it is cooled and drawn off as a separation product containing the thermolabile substance.

14. Method according to Claim 13, wherein the residence time of the film stream in the vertical film evaporator is from 1 to 100 seconds.

15. Method according to Claim 13, wherein the separation of a mixture of di- or polyamines or di- or polyisocyanates is carried out.
